(19) **Europäisches Patentamt European Patent Office Office européen des brevets**

(11) **EP 2 465 560 B1**

(12) **EUROPEAN PATENT SPECIFICATION**

(45) Date of publication and mention of the grant of the patent:
**08.08.2018 Bulletin 2018/32**

(51) Int Cl.:
*A61B 5/087* (2006.01)      *A61B 5/091* (2006.01)
*A61B 5/0488* (2006.01)      *A61M 16/00* (2006.01)

(21) Application number: **11170467.2**

(22) Date of filing: **19.06.2011**

(54) **Apparatus for the automatic control of ventilation phases based on pneumatic and non-pneumatic signals**

Apparat zur automatischen Steuerung der Beatmungsphasen basierend auf pneumatischen und nicht pneumatischen Signalen

Appareil de commande automatique des phases de ventilation à partir de signaux pneumatiques et non pneumatiques

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2010 DE 102010055253**

(43) Date of publication of application:
**20.06.2012 Bulletin 2012/25**

(73) Proprietor: **Drägerwerk AG & Co. KGaA**
**23558 Lübeck (DE)**

(72) Inventors:
• **Eger, Marcus**
**23562 Lübeck (DE)**

• **Hansmann, Hans-Ullrich**
**23858 Barnitz (DE)**
• **Glaw, Tobias**
**23558 Lübeck (DE)**
• **Sattler, Frank**
**23560 Lübeck (DE)**

(74) Representative: **Haseltine Lake LLP**
**Redcliff Quay**
**120 Redcliff Street**
**Bristol BS1 6HU (GB)**

(56) References cited:
**WO-A1-99/43374      WO-A1-2008/131798**
**US-A- 5 820 560**

## Description

The invention relates to an automatic control of a ventilator machine for changing over (triggering) between successive alternating phases of ventilation (inspiration and expiration phases).

The aim of the artificial maintaining of respiration with ventilator machines is to relieve the strain on a patient's respiratory muscles and to ensure that there is an adequate supply of oxygen and that carbon dioxide is eliminated to an adequate degree. This can be done by causing the ventilator machine to assume responsibility for the whole of the breathing activity or, in assisting techniques, for part of the breathing activity, existing breathing activity by the patient being assisted or boosted in these latter assisting techniques. For this purpose, the ventilator machines contain a ventilator unit or a supply of pressurised gas, to supply gas for breathing at a pressure which is preset by a control unit. Also present are sensors which sense, as a function of time, the pneumatic breathing signals, such for example as the airway pressure, or the flow of the gas for breathing and its volume (which is obtained by integrating the flow), and pass these signals on to the control unit.

In view of the increase in chronic lung disease and the demand for an improved therapy, the non-invasive assistance of breathing with improved interaction between the patient and the ventilator unit is a crucial requirement which needs to be met by modern-day ventilator machines. A significant object to be achieved in this case is the establishing of temporal synchrony between the assistance provided by the machine and the patient's own breathing activity. In the past, what was often done was to sedate patients who were breathing spontaneously to allow the ventilation to be correctly set and to allow synchrony to be forced to exist between the patient and the ventilator machine. From the knowledge which now exists, this procedure is no longer acceptable because risks have to be run of the lungs being damaged by the ventilation.

For improved synchronisation between the patient's breathing activity and the action of the ventilator unit, it is important for the beginning of inspiration and the beginning of expiration to be reliably detected in the patient's breathing activity. Especially in the case of neonates and COPD patients, the detection of phases of ventilation is often incorrect with conventional methods and results in increased breathing effort even going as far as exhaustion.

For artificial maintaining of respiration which is intended to take account of the patient's breathing activity in an improved way, it is known from DE 10 2007 062 214 B3 not only for pneumatic signals for breathing activity to be sensed but also for electromyographic signals to be picked up by electrodes on the thorax and for electromyographic signals for breathing activity (EMG signals) to be derived from these. These EMG signals are independent of the pneumatic signals for breathing activity and thus constitute an independent source of information which can be used to sense the beginning of inspiration and expiration. However it is not uncommon for the EMG signals to have interference and disruptions, such for example as the ECG signal from the heart, motion artefacts, or what is referred to as crosstalk (muscle activity which has nothing to do with the patient's respiratory system), superimposed on them.

For the last reason mentioned, EMG signals cannot readily be used as the sole basis for detecting the beginning of inspiration and expiration and for controlling the ventilator machine in the appropriate way. In this connection, there does exist a known changeover between signal sources in which, as dictated by signal quality, a changeover is made between pneumatic signals for breathing activity which are conventionally used (generally flow or pressure) and EMG signals, the signal quality being sensed by for example determining a signal to noise ratio. A changeover of this kind between the control of ventilation on the basis of EMG signals and control of ventilation on the basis of signals of other kinds is described in WO 2008/131798 A1. In this case a changeover is made between control on the basis of EMG signals to control on the basis of other signals for breathing activity if there is found to be a lack of synchrony between the EMG signal and breathing activity. Once there is again found to be adequate synchrony between the EMG signal and breathing activity, the ventilator machine reverts to the mode of operation on the basis of EMG signals. However, when there is a changeover of this kind between two signal sources, the contribution from one signal source is, basically, completely ignored, and independent information is wasted as a result. This is particularly disadvantageous when the quality of both signal sources is low.

Triggering of breaths on the basis of EMG signals is described in US 6,588,423 B1. In this case the raw EMG signal is pre-processed and, for triggering, a measure of the intensity of the EMG signal (the root mean square) is finally checked, the exceeding of a fixed threshold being used as a threshold criterion.

It is an object of the present invention to specify apparatus for the automatic control of a ventilator machine for changing over to the next phase of ventilation in a ventilator machine in which there is a more sensitive and earlier reaction to effort in breathing, without a transition to the next phase of ventilation being incorrectly triggered to an increased degree in the event of interference.

What is used to achieve this object is an apparatus which has the features given in claim 1. From WO 99/43374, there is known apparatus according to the preamble of claim 1.

Advantageous embodiments of the invention are specified in the dependent claims. In accordance with the present invention, a pneumatic signal for breathing activity $U_{pneu}(t)$ and a non-pneumatic signal for breathing activity $u_{non-pneu}(t)$ from a patient are sensed. What is meant by a pneumatic signal for breathing activity in the present case is a measurement signal which relates directly to the pneumatic control of ventilation, such for example as pressure, flow or volume. As well as this, a non-pneumatic signal for breathing activity is also sensed which, at the patient, represents the state of the respiratory system and breathing activity, such for example as an electromyographic signal (EMG signal) which is picked up from electrodes on the thorax, or the respiratory muscle pressure which is calculated by means of a model.

For these two independent signals, the respective distances $\Delta_{pneu}(t)$ and $\Delta_{non-pneu}(t)$ from associated threshold values which indicate the transition to the next phase of ventilation are determined as from a given reference time in the phase of ventilation (e.g. 200 ms after the beginning of the phase of ventilation). These distances from the associated threshold values are then standardised in relation to one another in such a way that the two distances can be directly compared with one another, which is achieved by standardising them to become equal distance values at the reference time; illustrative formulas for this purpose are given below. In this way the two distances $\Delta_{pneu}(t)$ and $\Delta_{non-pneu}(t)$ are standardised to equal values, to become $\delta_{pneu}(t)$ and $\delta_{non-pneu}(t)$, at the reference time. A mean is finally formed of these standardised distances from the threshold values or they are combined, to give a mean distance, and the changeover to the next phase of ventilation is made when the combined distance is 0.

The use of the size of the combined distance to detect the beginning of inspiration and expiration is distinctive for its greater robustness with, at the same time, faster detection. In this way it is possible to derive a triggering or control signal for the ventilator machine which, with the minimum necessary delay, represents the greatest possible amount of information on the activity of the patient and therefore allows more reliable support to be provided for the ventilation than was possible previously. In the prior art, different criteria for triggering the phases of ventilation have been combined with one another only insofar as the criterion which, in time, is met first has been given priority and has then triggered the changeover (first come, first served), i.e. a comparison with a threshold value has been carried out for each signal and the beginning of the phase of ventilation has been triggered as soon as one criterion has been met. This procedure is disadvantageous because no consideration is given to the reliability of the signals for breathing activity in comparison with their threshold values. It was therefore possible in the prior art for the threshold-value criterion which was met first in time to cause premature triggering of the beginning of the phase of ventilation, even though the signal for breathing activity which caused the triggering was possibly not very reliable and was possibly disrupted by artefacts.

By the combining in accordance with the invention of the distances from the threshold values of a pneumatic signal for breathing activity and a non-pneumatic signal for breathing activity, the amount of information, and hence the reliability of the changeover to the next phase of ventilation, is considerably increased because at least two signals for breathing activity which are substantially uncorrelated (i.e. interference in one signal generally does not occur in the other signal) go to make up the combined distance. Whereas pneumatic signals for breathing activity are correlated with one another to a greater or lesser degree (interference and disruptions affect all pneumatic signals in the same way), by the addition of an non-pneumatic signal for breathing activity it becomes possible to consult an independent source of information, which considerably improves the reliability with which the beginning of the phase of ventilation is correctly detected. If for example one of the two signals for breathing activity suffers interference such that the reaching of the associated threshold value is simulated, the second signal for breathing activity, which is uncorrelated with the first, will as a rule still be a considerable distance away from its threshold value, and the combined distance will thus still be sufficiently far away from 0 and a premature beginning of the next phase of ventilation will thus not be triggered.

The purpose of the reference time is to make provision for a minimum delay relative to the beginning of the current phase of ventilation. This delay needs to be sufficiently short for the likelihood that a fresh change to the next inspiration phase might already occur within this delay after the beginning of one phase of ventilation is vanishingly small. This is the case with a reference time of 200 ms. For the change to the next phase of ventilation then to be triggered at a later point in time, all that then needs to be done is for the standardised distances from the threshold values to be determined as from this reference time, and for these to be processed and observed.

The pneumatic signal for breathing activity is preferably selected from the signals for airway pressure, flow or volume. The non-pneumatic signal for breathing activity is selected from signals which, at the patient, represent the state of the respiratory system and breathing activity. Apart from the calculated respiratory muscle pressure, the measured oesophageal pressure and the gastric pressure, these signals are electrical signals, namely EMG signals, MMG signals (mechanomyographic signals), thorax impedance signals, fEIT signals, geometrical signals, namely thorax band signals, abdominal band signals and signals from strain gauges, movement-related signals, namely signals from acceleration sensors, and acoustic signals, namely signals from microphones. These non-pneumatic signals for breathing activity thus relate to the internal conditions of pressure within the patient which are connected with breathing activity, to electrical activity which is connected with breathing activity, to the geometrical change in size in the region of the thorax which is connected with breathing activity, to the movement in the region of the thorax which is connected with breathing activity, or to the acoustic activity which is connected with breathing activity. These signals for breathing activity are not correlated with the pneumatic signals for breathing activity which are sensed by the ventilator machine in the sense that an artefact or inference in one signal also results in one in the other signal. Due to this independence between the pneumatic and non-pneumatic signals for breathing activity, a large amount of information becomes available when the distances from the threshold values are being analysed, thus enabling the changeover to the beginning of the next phase of ventilation to be carried out with greater sensitivity but not, at the same time, with a greater susceptibility to interference or disruptions.

For the pneumatic signal for breathing activity, the standardised distance from the threshold value may be determined from for example

$$\delta_{pneu}(t) = \frac{u_{pneu}(t) - u_{pneu,thresh}(t)}{u_{pneu\,ref} - u_{pneu,thresh}(t)}$$

where $u_{pneu\,ref}$ is the value of the pneumatic signal for breathing activity at a preset reference time in the current phase of ventilation. At the reference time in the current phase of ventilation, the numerator and denominator are equal and the standardised distance at the reference time is 1.

In the above formula, the threshold $u_{pneu,thresh}(t)$ is shown as dependent on time; although a threshold which is constant over time can also be used, it is generally equally possible for what are referred to as dynamic thresholds to be used which vary as a function of time and which will be described below.

For the non-pneumatic signal for breathing activity, the standardised distance from the threshold value may be determined from for example

$$\delta_{non-pneu}(t) = \frac{u_{non-pneu}(t) - u_{non-pneu,thresh}(t)}{u_{non-pneu,ref} - u_{non-pneu,thresh}(t)}$$

where $u_{non-pneu,\,ref}$ is the non-pneumatic signal for breathing activity at the preset reference time after the beginning of the phase of ventilation.

What is preferably used as the pneumatic signal for breathing activity is the flow signal $\dot{V}(t)$, which means that the standardised distance from the associated threshold value is determined from

$$\delta\dot{V}(t) = \frac{\dot{V}(t) - \dot{V}_{thresh}(t)}{\dot{V}_{ref} - \dot{V}_{thresh}(t)}$$

where $\dot{V}_{ref}$ is the value of the flow signal at the preset reference time.

What is preferably used as the non-pneumatic signal for breathing activity is an electromyographic signal EMG(t), which means that the standardised distance from the associated threshold value is determined from

$$\delta\text{EMG}(t) = \frac{\text{EMG}(t) - \text{EMG}_{thresh}(t)}{\text{EMG}_{ref} - \text{EMG}_{thresh}(t)}$$

where $\text{EMG}_{ref}$ is the value of the EMG signal at the preset reference time in the phase of ventilation.

It can be seen that the standardisation of the distances from the associated threshold values is such that, at the reference time in the previous phase of ventilation, the distances of the non-pneumatic signal for breathing activity and the pneumatic signal for breathing activity are the same, namely are 1 in the present case.

To form a mean of the distances of the pneumatic and non-pneumatic signals for breathing activity from their associated threshold values, the means concerned can be determined, by the forming of a weighted mean, as a weighted arithmetic, geometrical or harmonic mean, as a weighted median or as a maximum or minimum.

For the forming of a weighted mean to determine the combined distance, quality indicators for the signals for breathing activity may be used, in the following way for example

$$K(t) = \frac{Q_{non-pneu}}{Q_{non-pneu} + Q_{pneu}} \cdot \delta_{non-pneu}(t) + \frac{Q_{pneu}}{Q_{non-pneu} + Q_{pneu}} \cdot \delta_{pneu}(t)$$

where $Q_{pneu}$ is a quality indicator for the pneumatic signal for breathing activity and $Q_{non-pneu}$ is a quality indicator for the non-pneumatic signal for breathing activity. Examples of quality indicators are given below.

The EMG signal used is preferably a pre-processed one. Pre-processing of this kind of the EMG signal is performed in a known manner in such a way that the raw EMG signal is freed of interfering and disrupting signals (e.g. ECG, motion artefacts, mains hum), and finally, envelope curve detection is carried out. Envelope curve detection may for example be performed by "rectification" followed by low pass filtering, the "rectification" being performed by an operation which gives a value (e.g. squaring or pure absolute-value generation). After the low pass filtering, what is then obtained is the envelope curve, i.e. the curve which is an envelope enclosing the waveform of the raw signal. A preferred implementation of the envelope curve detection process is the formation of what is referred to as the RMS (root mean square) over the length of a sliding time slot. The concept of EMG amplitude estimation which can be described by the term "envelope curve detection" is described in detail in Merletti R, Parker P.A.: Electromyography, Physiology, Engineering and Noninvasive Applications, IEEE Press, Wiley Interscience, 2004, Chapter 6.4 et seq, i.e. page 139 ff.

The invention will now be described, by way of example, only, by reference to the accompanying drawings, in which:

Fig. 1 is a graph which shows a pneumatic signal for breathing activity, namely flow, as a function of time over an expiration, a complete inspiration and the beginning of a further expiration,

Fig. 2 is a graph which shows a standardised distance from its threshold value for the pneumatic signal for breathing activity shown in Fig. 1,

Fig. 3 is a graph which shows a non-pneumatic signal for breathing activity, namely an EMG signal, as a function of time over the same period of time as in Fig. 1,

Fig. 4 is a graph which shows a standardised distance from its threshold value for the non-pneumatic signal for breathing activity shown in Fig. 3, and

Fig. 5 is a graph which shows the standardised distances shown in Figs. 2 and 4, and a mean distance formed therefrom, as a function of time over an expiration phase.

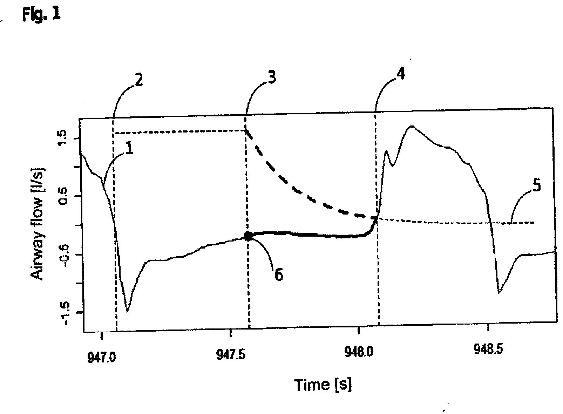

What is shown in Fig. 1 as a pneumatic signal for breathing activity is flow as a function of time for a period which shows the end of an inspiration, a complete expiration which succeeds the latter, a complete inspiration which succeeds this expiration and the beginning of the next expiration. The flow signal has been given the reference numeral 1. At time 2, there is a changeover to the expiration phase as a result of the meeting of criteria which have yet to be described. At this time there is also a resetting of the threshold value which will be used for the changeover to the next inspiration phase. In the present embodiment, what is used is not a fixed threshold value (which would be a horizontal straight line in Fig. 1), but what is referred to as a dynamic threshold. In the present case, what is suitable for detecting the next inspiration is a dynamic threshold which first begins, at the start of an expiration, at a relatively high value. This relatively high value lasts for a preset period of time and the dynamic threshold is then reduced to a target value which should be reached at the time when the expiration is expected to end; the expected end point of the expiration may for example be estimated from the duration of the previous expiration phase. The preset period of time for which the threshold is held at a relatively high constant value after the beginning of the expiration is to be selected to be such that it is appreciably shorter than all the expected expiration phases, i.e. the likelihood of a new inspiration beginning as early as during this period of time should be vanishingly small; a period of time of this kind may for example be 200 ms. The target value for the threshold may for example be derived from the maximum and minimum values $u_{pneu}^{max}$, $u_{pneu}^{min}$, $u_{non-pneu}^{max}$ and $u_{non-pneu}^{min}$ of the signals in previous phases of ventilation.

Conversely, the threshold for detecting the next expiration begins, at the start of an inspiration, at a low value which lasts for a preset period of time and the threshold is then raised to a preset target value which should be reached at the time when the inspiration is expected to end. The expected end point of the inspiration may, in turn, be estimated from the duration of the previous inspiration phases and the target values may be derived from the maximum and minimum values $u_{pneu}^{max}$, $u_{pneu}^{min}$, $u_{non-pneu}^{max}$ and $u_{non-pneu}^{min}$ of the signals in previous phases of ventilation.

The consideration underlying a dynamic threshold of this kind is that it is precisely at the beginning of a phase of ventilation that there is a very small likelihood that a change to the next phase of ventilation might again take place as early as within the short period of time which is preset. It is therefore possible to use, at the beginning of a phase of ventilation, thresholds which are set in such a way that the possibility of an erroneous changeover can be virtually ruled out even if there is severe interference or disruption, whereas after the preset period of time the threshold is moved to a target value which is to be reached at the expected end of the phase of ventilation, thus enabling the beginning of the next phase of ventilation to be detected with great sensitivity. The expected value may correspond to the value which would be suitable for a sensitive constant threshold. This being the case, there is an assurance of sensitive triggering towards the end of the phase of ventilation but incorrect triggering as a result of interference or disruptions is suppressed at the beginning of the phase.

If triggering only took place in response to the pneumatic signal for breathing activity, the next inspiration phase would be triggered by the signal for breathing activity 1 on the threshold 5 being reached at time 4. However, in accordance with the present invention, what is used is not a threshold criterion referred to a single signal but combined threshold criteria which are based on different signals for breathing activity.

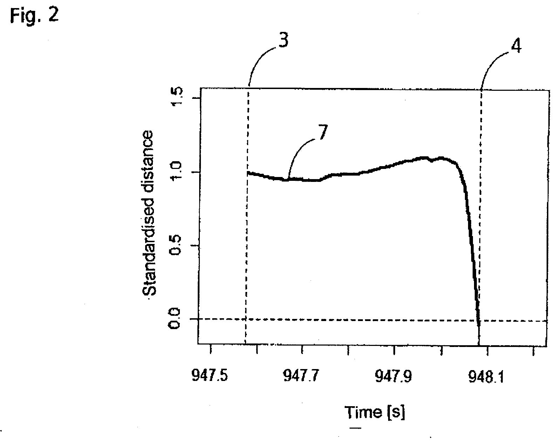

In Fig. 2, the distance between the flow signal 1 and the dynamic threshold 5 from Fig. 1, after standardisation as a standardised distance $\delta_{pneu}(t)$, is plotted (7) over an expiration phase. The distance $\delta_{pneu}(t)$ is determined as from the reference time in the phase of ventilation. The standardisation is performed in this case in such a way that the existing distance between the flow signal 1 and the dynamic threshold 5 is standardised to the same value, namely 1, by means of the distance between the value of the flow signal and the dynamic threshold at the preset reference time in the current phase of ventilation:

$$\delta_{pneu}(t) = \frac{u_{pneu}(t) - u_{pneu,thresh}(t)}{u_{pneu,ref} - u_{pneu,thresh}(t)}$$

In Fig. 1, a preset reference time of this kind is identified as 3 and the corresponding value of the flow signal is identified as 6. The pneumatic and non-pneumatic distances are standardised to be of the same value at this time.

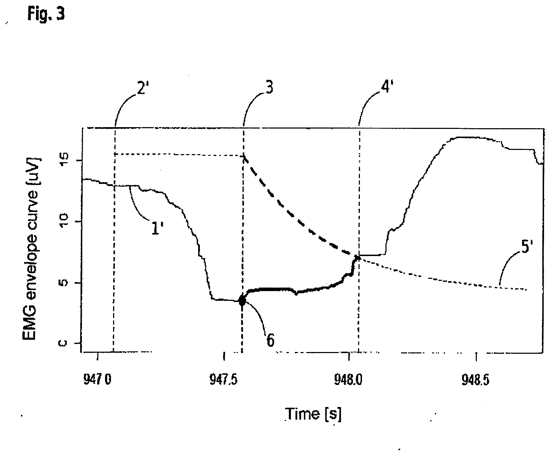

In Fig. 3, the non-pneumatic signal for breathing activity is plotted as a function of time for the same length of time as in Fig. 1. The EMG signal is identified as 1'. The beginning of the expiration phase is shown as 2 and 3 indicates the short preset period of time following the beginning of the expiration phase 2 after which the initially high and constant dynamic threshold 5' is lowered.

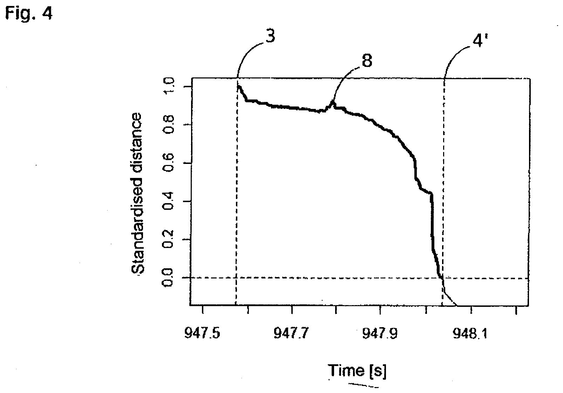

The standardised distance for the EMG signal shown in Fig. 3 is shown in Fig. 4. As was explained above in relation to Fig. 2, the standardised distance begins with a value of 1 at the reference time 3. The scales for the standardised distances are thus the same for the pneumatic signal for breathing activity shown in Fig. 2 and for the non-pneumatic signal for breathing activity shown in Fig. 4 and this means that they can be compared and combined with one another in a suitable way.

The standardised distance 8 for the non-pneumatic EMG signal shown in Fig.4 reaches a value of 0 at time 4'. This would thus be the triggering time if it were only the EMG signal which were used for the changeover of the phases of ventilation.

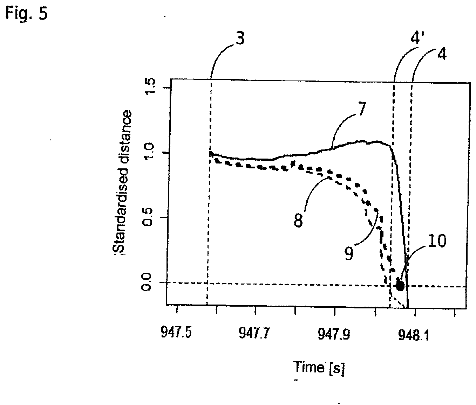

In Fig. 5, the standardised distances 5 and 6 from Figs. 2 and 4 are combined in a single plot. It can be seen that the distances do not become 0 at the same time and that in the present case the distance 8 for the EMG signal reaches a value of 0 at an earlier time 4' than the distance 7 for the flow signal reaches the value of 0, which it does at time 4. In conventional methods, it would have been decided in advance which signal for breathing activity was to supply the trigger for the changeover of the phases of ventilation, meaning that the changeover from expiration to inspiration would have taken place either at time 4' (if the EMG signal had been used as a trigger) or at time 4 (if the flow signal had been used to trigger the next inspiration).

In accordance with the present invention, a mean is formed of the two distances or they are combined, to give a mean distance. A simple arithmetic mean may be formed when this is done but there are also other means which can be used such as, say, a geometrical or harmonic mean, a median or a maximum or minimum. What is preferred however is not for a simple arithmetic mean to be formed but for a weighted mean to be formed. When this is done, the weighting should weight more highly that distance which corresponds to whichever one it is of the pneumatic and non-pneumatic signals for breathing activity which is of the higher quality as a signal. A formula which expresses the formation of a mean of this kind using weighting factors based on signal quality may then be as follows:

$$K(t) = \frac{Q_{non-pneu}}{Q_{non-pneu} + Q_{pneu}} \cdot \delta_{non-pneu}(t) + \frac{Q_{pneu}}{Q_{non-pneu} + Q_{pneu}} \cdot \delta_{pneu}(t)$$

where $Q_{pneu}$ is a quality indicator for the pneumatic signal for breathing activity and $Q_{non-pneu}$ is a quality indicator for the non-pneumatic signal for breathing activity. The formation of a weighted mean of this kind based on signal quality was carried out in the example shown in Fig. 5, this having resulted in the present case in a weighting of 80% for the EMG signal and one of 20% for the flow signal. Amongst other things, this results in the passage through zero at 8 for the combined distance being closer to the passage through zero at 4' for the EMG signal than to the passage through zero at 4 for the flow signal.

What may be selected as a quality indicator for the flow signal is for example:

$$Q_{flow} = max\{0.1-V_{leak}/V_{exp}\}.$$

In this equation, $V_{leak}$ is the volume which flows out through a leak. When there is a minimal leak, the value of this quality indicator is close to 1. When there is a larger leak which results in a $V_{leak}$ close to the expiratory volume, the quality indicator approaches a value of 0. There are also alternative quality indicators which can be defined which take account of, for example, the disruptions caused by condensation in the breathing tube system or cardiogenic oscillations. This is possible by, for example, determining the signal powers in higher frequency ranges by Fourier transformation and finding a relationship between them and a reference power (such for example as the signal power in the frequency range of respiration).

What suggests themselves as quality indicators for the EMG envelope curve signal are the degree of suppression of artefacts (e.g. QRS complexes, i.e. deflections and peaks in the ECG signal), and the ratio of the mean activity of the signal during inspiration to the mean activity during expiration.

The degree of elimination or suppression of artefacts - specifically QRS complexes in the present case - can be determined for example in the form of a power ratio $Q_{QRS} = min\{1, P_{mean}/P_{QRS}\}$. $P_{QRS}$ is the mean signal power in past time slots which included QRS complexes. $P_{mean}$ on the other hand is the total mean power. The mean powers can be calculated for example as RMS values of the signal divided by the length of the interval of time over which the RMS calculation is made. $Q_{QRS}$ is 1 when $P_{QRS}$ is not greater than the mean power $P_{mean}$, which means that the artefacts no longer make any, or hardly any, contribution to the mean activity, i.e. they are sufficiently eliminated. If on the other hand $P_{QRS}$ is considerably higher than the mean power $P_{mean}$, the value of the indicator $Q_{QRS}$ tends towards 0.

Alternatively, the total mean power $P_{mean}$ can be compared with the power during inspiration $P_{insp}$. The corresponding quality indicator is then $Q_{insp} = max\{0.1-P_{mean}/P_{insp}\}$. $Q_{insp}$ tends towards 1 when the power during inspiration $P_{insp}$ is considerably higher than the mean power $P_{mean}$. Conversely, $Q_{insp}$ reaches a value of 0 when the power during inspiration $P_{insp}$ is not higher than the mean power $P_{mean}$.

Similarly, the total mean power $P_{mean}$ can be compared with the power during expiration $P_{exp}$. The quality indicator is then $Q_{exp} = max\{0.1-P_{mean}/P_{exp}\}$. $Q_{insp}$ reaches a value of 1 when the power during expiration $P_{exp}$ is considerably higher than the mean power $P_{mean}$.

These quality indicators quantify the ability of inspiration or expiration effort to be differentiated from mean activity and constitute a kind of standardised signal to noise ratio. Only when there is a good ability to be differentiated, i.e. when the mean inspiration activity is higher, or the mean expiration activity is lower, than the mean activity is it advisable for an EMG envelope curve signal of this kind to be used for triggering.

A prerequisite for the valid determination of a quality indicator of this kind is that the phases of breathing are clearly determined, by for example an analysis of the pneumatic signals, because it is only then that the time slots for determining the powers are known. What suggests itself in practice is for the mean powers of the EMG envelope curve signal to be determined at a CPAP ventilation setting, i.e. with assisting breaths omitted. This prevents possible asynchrony, or even excessively severe suppression of the EMG activity as a result of a high level of assistance, from occurring. The omission of individual assisting breaths can be accomplished by means of transient actions at the ventilation machine.

To obtain a quality indicator for the EMG envelope curve signal, provision may for example be made for the use of a combination of the quality indicators mentioned above, in the form of a product for example

$$Q_{emg} = Q_{insp} \cdot (1-Q_{exp}) \cdot Q_{QRS}.$$

$Q_{emg}$ reaches a value of 1 when the inspiratory activity shown by the EMG envelope curve signal is high and the expiratory activity low in comparison with the mean activity and the QRS artefacts have been successfully eliminated. The above condition of the inspiratory activity being high and the expiratory activity low in comparison with the mean activity means that inspiration with increased muscular activity when there is an effort to breathe is reflected by increased signal activity, whereas this in not the case in expiration, which is consistent with the assumption of the EMG envelope curve signal being a true reflection of muscular activity.

As an alternative to the formation of a product described above, the individual quality indicators may also be combined by the forming of a weighted mean.

$$Q_{emg} = \alpha_1 Q_{insp} + \alpha_2(1-Q_{exp}) + \alpha_3 Q_{QRS}.$$

where the sum of the weights $\alpha_i$ gives a value of 1. If for example this combined quality indicator is used for assessing the inspiratory triggering criteria, it is possible that the effect of $Q_{insp}$ may have to be considered more important than than of $Q_{exp}$ because increased expiratory activity is not very relevant to inspiratory triggering. In view of this, $\alpha_1$ might be selected to be considerably higher than $\alpha_2$.

It is also possible for quality indicators to be defined which assign a rating to the variability of the breathing cycles. This can for example be achieved by determining the variability of the lengths of past breaths (e.g. by calculating the standard deviation) in relation to the mean length of a breath. Whereas a very low variability appears to be pathological, a very high variability is an indication of asynchrony and increasing effort in breathing, possibly as a result of an incorrect ventilation setting.

Other possible quality indicators are based on the coefficient of correlation, the correlation function, transinformation (mutual information) or on some other statistical measure for determining properties of correlation between different respiratory signals. As an example, the coefficient of correlation between the flow signal and a signal from a strain gauge (fastened to the thorax) would have to be very high because both signals would be significant representations of the change in the volume of the thorax. If only a low value is found for the coefficient of correlation, either the flow signal or the signal from the strain gauge (or both of them) is subject to interference or disruption. In the event of the flow signal for example being estimated to be reliable by some other means, it has to be assumed that the quality of the signal from the strain gauge is low.

Other possible suitable ways of combining quality indicators of this kind are, as described above, a weighted arithmetic mean but also a geometrical or harmonic means (weighted if required) and also a median (weighted if required).

The quality indicators may be quantised, i.e. divided into ranges (e.g. into categories such as low = [0 - 0.3], medium = [0.3 - 0.7] and high = [0.7 - 1]) before they are combined. The quality indicators may also be subjected to switching in response to a threshold value so that, for example, a quality indicator does not effectively become part of the combination until it has exceeded a minimum value.

What also suggests itself for analysing the combination of quality indicators is the use of a fuzzy controller, particularly when what are to be combined are a large number of quality indicators and other possible influencing factors, which may even contradict one another. The use of fuzzy controllers is part of the prior art and there will be no further description of it here.

## Claims

1. Apparatus for the automatic control of a ventilator machine for changing over (triggering) between successive phases of ventilation (inspiration and expiration phases), said apparatus determining, from a pneumatic signal for breathing activity $U_{pneu}(t)$ (1) and a non-pneumatic signal for breathing activity $u_{non\text{-}pneu}(t)$ (1') from a patient, as from a preset reference time (3) after the beginning of a phase of ventilation, a distance $\Delta_{pneu}(t)$ of the pneumatic signal for breathing activity $U_{pneu}(t)$ (1) from an associated threshold value $u_{pneu,thresh}(t)$ (5) and a distance $A_{non\text{-}pneu}(t)$ of the non-pneumatic signal for breathing activity $u_{non\text{-}pneu}(t)$ (1') from an associated threshold value $u_{non\text{-}pneu,thresh}(t)$ (5'), **characterised by** the apparatus which is configured to standardise these distances $\Delta_{pneu}(t)$ and $A_{non\text{-}pneu}(t)$ in relation to one another, to become $\delta_{pneu}(t)$ (7) and $\delta_{non\text{-}pneu}(t)$ (8), in such a way that their values are the same at the preset reference time (3), the apparatus is further configured to combine the standardised distances $\delta_{pneu}(t)$ (7) and $\delta_{non\text{-}pneu}(t)$ (8) to give a mean distance, and the apparatus is configured to control a changeover to the next phase of ventilation when the combined distance is 0.

2. The apparatus according to claim 1, in which the pneumatic signal for breathing activity $U_{pneu}(t)$ is selected from airway pressure, flow or volume.

3. The apparatus according to either of the preceding claims, in which the non-pneumatic signal for breathing activity $u_{non\text{-}pneu}(t)$ is selected from respiratory muscle pressure, oesophageal pressure or gastric pressure, from electrical signals, namely EMG signals, MMG signals, thorax impedance signals, fEIT signals, geometrical signals, namely thorax band signals, abdominal band signals and signals from strain gauges, movement-related signals, namely signals from acceleration sensors, or acoustic signals, namely signals from microphones.

4. The apparatus according to any one of the preceding claims, in which, for the pneumatic signal for breathing activity, the standardised distance from the threshold value is determined from

$$\delta_{pneu}(t) = \frac{u_{pneu}(t) - u_{pneu,thresh}(t)}{u_{pneu\,ref} - u_{pneu,thresh}(t)}$$

where $u_{pneu\,ref}$ is the pneumatic signal for breathing activity at the preset reference time in the current phase of ventilation.

5. The apparatus according to any one of the preceding claims, in which, for the non-pneumatic signal for breathing activity, the standardised distance from the threshold value is determined from

$$\delta_{non-pneu}(t) = \frac{u_{non-pneu}(t) - u_{non-pneu,thresh}(t)}{u_{non-pneu,ref} - u_{non-pneu,thresh}(t)}$$

where $u_{non-pneu\,ref}$ is the non-pneumatic signal for breathing activity at the preset reference time in the current phase of ventilation.

6. The apparatus according to any one of the preceding claims, in which the flow signal $\dot{V}(t)$ is used as the pneumatic signal for breathing activity and the standardised distance from the associated threshold value is determined from

$$\delta\dot{V}(t) = \frac{\dot{V}(t) - \dot{V}_{thresh}(t)}{\dot{V}_{ref} - \dot{V}_{thresh}(t)}$$

where $\dot{V}_{ref}$ is the value of the flow signal at the preset reference time in the current phase of ventilation.

7. The apparatus according to any one of the preceding claims, in which an electromyographic signal EMG(t) is used as the non-pneumatic signal for breathing activity and the standardised distance from the associated threshold value is determined from

$$\delta\mathrm{EMG}(t) = \frac{\mathrm{EMG}(t) - EMG_{thresh}(t)}{\mathrm{EMG}_{ref} - \mathrm{EMG}_{thresh}(t)}$$

where $\mathrm{EMG}_{ref}$ is the EMG signal at the preset reference time in the current phase of ventilation.

8. The apparatus according to any one of the preceding claims, in which the combined distance is formed from the standardised distances $\delta_{pneu}(t)$ and $\delta_{non-pneu}(t)$ in the form of a weighted arithmetic, geometrical or harmonic mean, a weighted median or a maximum or minimum.

9. The apparatus according to claim 8, in which the combined distance is determined from the equation:

$$K(t) = \frac{Q_{non-pneu}}{Q_{non-pneu} + Q_{pneu}} \cdot \delta_{non-pneu}(t) + \frac{Q_{pneu}}{Q_{non-pneu} + Q_{pneu}} \cdot \delta_{pneu}(t)$$

where $Q_{pneu}$ is a quality indicator for the pneumatic signal for breathing activity and $Q_{non-pneu}$ is a quality indicator for the non-pneumatic signal for breathing activity.

10. The apparatus according to any one of the preceding claims, in which a plurality of non-pneumatic signals for breathing activity $u_{non-pneu,\,i}(t)$ (i = 1, ... n) from a patient are sensed and the distance $\Delta_{non-pneu,\,i}(t)$ and the standardised distance $\delta_{non-pneu,i}(t)$ are determined for each of them and a mean is found of all the distances $\delta_{pneu}(t)$ and $\delta_{non-pneu,i}(t)$ to give a mean distance and a changeover is made to the next phase of ventilation when the combined distance is 0.

**11.** The apparatus according to claim 10, in which the combined distance is determined from the equation:

$$K(t) = \sum_{i=1}^{n} \frac{Q_{non-pneu,i}}{\sum_{j=1}^{n} Q_{non-pneu,j} + Q_{pneu}} \cdot \delta_{non-pneu,i}(t) + \frac{Q_{pneu}}{\sum_{j=1}^{n} Q_{non-pneu,j} + Q_{pneu}} \cdot \delta_{pneu}(t),$$

where $Q_{pneu}$ is a quality indicator for the pneumatic signal for breathing activity and $Q_{non-pneu,i}$ is a quality indicator for the non-pneumatic signal for breathing activity $u_{non-pneu,i}(t)$ (i = 1, ... n).

**12.** The apparatus according to any preceding claim, comprising a ventilator machine having a ventilator unit or a controllable pressurised supply of gas for breathing, having a control unit which is arranged to control the ventilator unit or the pressurised supply of gas for breathing to carry out successive inspiration and expiration phases, and sensors, for sensing said pneumatic signal for breathing activity $u_{pneu}(t)$ and said non-pneumatic signal for breathing activity $u_{non-pneu}(t)$, being connected to the control unit to transmit their measurement signals.

**Patentansprüche**

**1.** Einrichtung für die automatische Steuerung einer Beatmungsmaschine zum Wechsel (Auslösen) zwischen aufeinanderfolgenden Phasen einer Beatmung (Einatmungs- und Ausatmungsphasen), wobei die Einrichtung aus einem pneumatischen Signal für eine Atmungsaktivität $u_{pneu}(t)$ (1) und einem nichtpneumatischen Signal für eine Atmungsaktivität $u_{non-pneu}(t)$ (1') von einem Patienten, wie von einer vorbestimmten Referenzzeit (3) nach dem Beginn einer Phase der Beatmung, einen Abstand $\Delta_{pneu}(t)$ des pneumatischen Signals für eine Atmungsaktivität $u_{pneu}(t)$ (1) von einem assoziierten Schwellenwert $u_{pneu,thresh}(t)$ (5) und einen Abstand $\Delta_{non-pneu}(t)$ des nichtpneumatischen Signals für eine Atmungsaktivität $u_{non-pneu}(t)$ (1') von einem assoziierten Schwellenwert $u_{non-pneu,thresh}(t)$ (5') bestimmt, **gekennzeichnet durch** die Einrichtung, die dazu konfiguriert ist, diese Abstände $\Delta_{pneu}(t)$ und $\Delta_{non-pneu}(t)$ relativ zueinander auf eine derartige Art zu standardisieren, damit sie zu $\delta_{pneu}(t)$ (7) und $\delta_{non-pneu}(t)$ (8) werden, sodass ihre Werte gleich der voreingestellten Referenzzeit (3) sind, wobei die Einrichtung ferner dazu konfiguriert ist, die standardisierten Abstände $\delta_{pneu}(t)$ (7) und $\delta_{non-pneu}(t)$ (8) zu kombinieren, um einen mittleren Abstand zu geben, und wobei die Einrichtung dazu konfiguriert ist, einen Wechsel zu der nächsten Phase einer Beatmung zu steuern, wenn der kombinierte Abstand 0 ist.

**2.** Einrichtung nach Anspruch 1, bei der das pneumatische Signal für eine Atmungsaktivität $u_{pneu}(t)$ aus einem Atemwegdruck, einem Durchfluss und einem Volumen ausgewählt wird.

**3.** Einrichtung nach einem der vorhergehenden Ansprüche, bei der das nichtpneumatische Signal für eine Atmungsaktivität $u_{non-pneu}(t)$ aus einem Atemmuskeldruck, einem Speiseröhrendruck oder einem Magendruck, aus elektrischen Signalen, nämlich EMG-Signalen, MMG-Signalen, Thoraximpedanzsignalen, fEIT-Signalen, geometrischen Signalen, nämlich Thoraxbandsignalen, Abdomenbandsignalen und Signalen von Dehnungsmessstreifen, bewegungsbezogenen Signalen, nämlich Signalen von Beschleunigungssensoren, oder akustischen Signalen, nämlich Signalen von Mikrofonen, ausgewählt wird.

**4.** Einrichtung nach einem der vorhergehenden Ansprüche, bei der der standardisierte Abstand von dem Schwellenwert für das pneumatische Signal für eine Atmungsaktivität bestimmt wird aus:

$$\delta_{pneu}(t) = \frac{u_{pneu}(t) - u_{pneu,thresh}(t)}{u_{pneu\ ref} - u_{pneu,thresh}(t)},$$

wobei $u_{pneu\ ref}$ das pneumatische Signal für eine Atmungsaktivität bei der voreingestellten Referenzzeit in der momentanen Phase einer Beatmung ist.

**5.** Einrichtung nach einem der vorhergehenden Ansprüche, bei der der standardisierte Abstand von dem Schwellenwert für das nichtpneumatische Signal für eine Atmungsaktivität bestimmt wird aus:

$$\delta_{non-pneu}(t) = \frac{u_{non-pneu}(t) - u_{non-pneu,thresh}(t)}{u_{non-pneu\ ref} - u_{non-pneu,thresh}(t)},$$

wobei $u_{non-pneu\ ref}$ das nichtpneumatische Signal für eine Atmungsaktivität bei der voreingestellten Referenzzeit in der momentanen Phase einer Beatmung ist.

6. Einrichtung nach einem der vorhergehenden Ansprüche, bei der das Durchflusssignal $\dot{V}(t)$ als das pneumatische Signal für eine Atmungsaktivität verwendet wird und der standardisierte Abstand von dem assoziierten Schwellenwert bestimmt wird aus:

$$\delta\dot{V}(t) = \frac{\dot{V}(t) - \dot{V}_{thresh}(t)}{\dot{V}_{ref} - \dot{V}_{thresh}(t)},$$

wobei $\dot{V}_{ref}$ der Wert des Flusssignals bei der voreingestellten Referenzzeit in der momentanen Phase einer Beatmung ist.

7. Einrichtung nach einem der vorhergehenden Ansprüche, bei der ein elektromyographisches Signal EMG(t) als das nichtpneumatische Signal für eine Atmungsaktivität verwendet wird und der standardisierte Abstand von dem assoziierten Schwellenwert bestimmt wird aus

$$\delta EMG(t) = \frac{EMG(t) - EMG_{thresh}(t)}{EMG_{ref} - EMG_{thresh}(t)},$$

wobei $EMG_{ref}$ das EMG-Signal bei der voreingestellten Referenzzeit in der momentanen Phase einer Beatmung ist.

8. Einrichtung nach einem der vorhergehenden Ansprüche, bei der der kombinierte Abstand aus den standardisierten Abständen $\delta_{pneu}(t)$ und $\delta_{non-pneu}(t)$ in der Form eines gewichteten arithmetischen, geometrischen oder harmonischen Mittels, eines gewichteten Medians oder eines Maximums oder Minimums gebildet wird.

9. Einrichtung nach Anspruch 8, bei der der kombinierte Abstand aus der folgenden Gleichung bestimmt wird:

$$K(t) = \frac{Q_{non-pneu}}{Q_{non-pneu} + Q_{pneu}} \cdot \delta_{non-pneu}(t) + \frac{Q_{pneu}}{Q_{non-pneu} + Q_{pneu}} \cdot \delta_{pneu}(t),$$

wobei $Q_{pneu}$ ein Qualitätsindikator für das pneumatische Signal für eine Atmungsaktivität ist und $Q_{non-pneu}$ ein Qualitätsindikator für das nichtpneumatische Signal für eine Atmungsaktivität ist.

10. Einrichtung nach einem der vorhergehenden Ansprüche, bei der mehrere nichtpneumatische Signale für eine Atmungsaktivität $u_{non-pneu,i}(t)$ (i=1, ..., n) von einem Patienten erfasst werden und der Abstand $\Delta_{non-pneu,i}(t)$ und der standardisierte Abstand $\delta_{non-pneu,i}(t)$ für jedes von ihnen bestimmt werden und ein Mittelwert sämtlicher Abstände $\delta_{pneu}(t)$ und $\delta_{non-pneu,i}(t)$ gefunden wird, um einen mittleren Abstand zu geben, und ein Wechsel zu der nächsten Phase einer Beatmung erfolgt, wenn der kombinierte Abstand 0 ist.

11. Einrichtung nach Anspruch 10, bei der der kombinierte Abstand aus der folgenden Gleichung bestimmt wird:

$$K(t) = \sum_{i=1}^{n} \frac{Q_{non-pneu,i}}{\sum_{j=1}^{n} Q_{non-pneu,j} + Q_{pneu}} \cdot \delta_{non-pneu,i}(t) + \frac{Q_{pneu}}{\sum_{j=1}^{n} Q_{non-pneu,j} + Q_{pneu}} \cdot$$
$$\delta_{pneu}(t),$$

wobei $Q_{pneu}$ ein Qualitätsindikator für das pneumatische Signal für eine Atmungsaktivität ist und $Q_{non-pneu,i}$ ein Qualitätsindikator für das nichtpneumatische Signal für eine Atmungsaktivität $u_{non-pneu,i}(t)$ (i=l, ..., n) ist.

12. Einrichtung nach einem vorhergehenden Anspruch, die eine Beatmungsmaschine mit einer Beatmungseinheit oder einer steuerbaren druckbeaufschlagten Versorgung eines Atemgases umfasst und die eine Steuereinheit, die zum Steuern der Beatmungseinheit oder der druckbeaufschlagten Versorgung eines Atemgases eingerichtet ist, um aufeinanderfolgende Einatmungs- und Ausatmungsphasen auszuführen, und Sensoren zum Erfassen des pneumatischen Signals für eine Atemaktivität $u_{pneu}(t)$ und des nichtpneumatischen Signals für eine Atemaktivität $u_{non-pneu}(t)$, die mit der Steuereinheit zum Übertragen ihrer Messsignale verbunden ist, aufweist.

**Revendications**

1. Appareil pour le contrôle automatique d'une machine de ventilation destinée à passer à (déclencher) des phases successives de ventilation (phases d'inspiration et d'expiration), ledit appareil déterminant, à partir d'un signal pneumatique pour l'activité de respiration $u_{pneu}(t)$ (1) et d'un signal non pneumatique pour l'activité de respiration $u_{non\ pneu}(t)$ (1') d'un patient, ainsi qu'à partir d'un temps de référence prédéfini (3) après le début d'une phase de ventilation, une distance $\Delta_{pneu}(t)$ du signal pneumatique pour l'activité de respiration $u_{pneu}(t)$ (1) par rapport à une valeur seuil associée $u_{pneu,seuil}(t)$ (5) et une distance $\Delta_{non\ pneu}(t)$ du signal non pneumatique pour l'activité de respiration $u_{non\ pneu}(t)$ (1') par rapport à une valeur seuil associée $u_{non\ pneu,seuil}(t)$ (5'), **caractérisé en ce que** l'appareil est configuré pour normaliser ces distances $\Delta_{pneu}(t)$ et $\Delta_{non\ pneu}(t)$ l'une par rapport à l'autre, pour qu'elles deviennent $\delta_{pneu}(t)$ (7) et $\delta_{non\ pneu}(t)$ (8), de manière à ce que leurs valeurs soient identiques au temps de référence prédéfini (3), l'appareil est également configuré pour combiner les distances normalisées $\delta_{pneu}(t)$ (7) et $\delta_{non\ pneu}(t)$ (8) pour obtenir une distance moyenne, et l'appareil est configuré pour contrôler un passage à la phase de ventilation suivante quand la distance combinée est égale à 0.

2. Appareil selon la revendication 1, dans lequel le signal pneumatique pour l'activité de respiration $u_{pneu}(t)$ est choisi parmi la pression, le débit ou le volume des voies aériennes.

3. Appareil selon l'une ou l'autre des revendications précédentes, dans lequel le signal non pneumatique pour l'activité de respiration $u_{non\ pneu}(t)$ est choisi parmi la pression musculaire respiratoire, la pression oesophagienne ou la pression gastrique, parmi des signaux électriques, à savoir des signaux EMG, des signaux MMG, des signaux d'impédance thoracique, des signaux fEIT, des signaux géométriques, à savoir des signaux de la bande thoracique, des signaux de la bande abdominale et des signaux provenant de jauges de contrainte, des signaux associés à un mouvement, à savoir des signaux provenant de capteurs d'accélération, ou des signaux acoustiques, à savoir des signaux provenant de microphones.

4. Appareil selon l'une quelconque des revendications précédentes, dans lequel, pour le signal pneumatique pour l'activité de respiration, la distance normalisée par rapport à la valeur seuil est déterminée à partir de

$$\delta_{pneu}(t) = \frac{u_{pneu}(t) - u_{pneu,seuil}(t)}{u_{pneu\ réf} - u_{pneu,seuil}(t)}$$

où $u_{pneu\ réf}$ est le signal pneumatique pour l'activité de respiration au temps de référence prédéfini dans la phase de ventilation en cours.

5. Appareil selon l'une quelconque des revendications précédentes, dans lequel, pour le signal non pneumatique pour l'activité de respiration, la distance normalisée par rapport à la valeur seuil est déterminée à partir de

$$\delta_{non\ pneu}(t) = \frac{u_{non\ pneu}(t) - u_{non\ pneu,seuil}(t)}{u_{non\ pneu\ réf} - u_{non\ pneu,seuil}(t)}$$

où $u_{non\ pneu\ réf}$ est le signal non pneumatique pour l'activité de respiration au temps de référence prédéfini dans la phase de ventilation en cours.

6. Appareil selon l'une quelconque des revendications précédentes, dans lequel le signal de débit $\dot{V}(t)$ est utilisé comme le signal pneumatique pour l'activité de respiration et la distance normalisée par rapport à la valeur seuil associée est déterminée à partir de

$$\delta\dot{V}(t) = \frac{\dot{V}(t) - \dot{V}_{seuil}(t)}{\dot{V}_{réf} - \dot{V}_{seuil}(t)}$$

où $\dot{V}_{réf}$ est la valeur du signal de débit au temps de référence prédéfini dans la phase de ventilation en cours.

**7.** Appareil selon l'une quelconque des revendications précédentes, dans lequel un signal électromyographique EMG(t) est utilisé comme le signal non pneumatique pour l'activité de respiration et la distance normalisée par rapport à la valeur seuil associée est déterminée à partir de

$$\delta EMG(t) = \frac{EMG(t) - EMG_{seuil}(t)}{EMG_{réf} - EMG_{seuil}(t)}$$

où $EMG_{réf}$ est le signal EMG au temps de référence prédéfini dans la phase de ventilation en cours.

**8.** Appareil selon l'une quelconque des revendications précédentes, dans lequel la distance combinée est formée à partir des distances normalisées $\delta_{pneu}(t)$ et $\delta_{non\ pneu}(t)$ sous la forme d'une moyenne arithmétique, géométrique ou harmonique pondérée, d'une médiane pondérée ou d'un maximum ou minimum.

**9.** Appareil selon la revendication 8, dans lequel la distance combinée est déterminée à partir de l'équation :

$$K(t) = \frac{Q_{non\ pneu}}{Q_{non\ pneu} + Q_{pneu}} \cdot \delta_{non\ pneu}(t) + \frac{Q_{pneu}}{Q_{non\ pneu} + Q_{pneu}} \cdot \delta_{pneu}(t)$$

où $Q_{pneu}$ est un indicateur de qualité pour le signal pneumatique pour l'activité de respiration et $Q_{non\ pneu}$ est un indicateur de qualité pour le signal non pneumatique pour l'activité de respiration.

**10.** Appareil selon l'une quelconque des revendications précédentes, dans lequel une pluralité de signaux non pneumatiques pour l'activité de respiration $u_{non\ pneu,i}(t)$ (i = 1, ..., n) d'un patient sont détectés et la distance $\Delta_{non\ pneu,i}(t)$ et la distance normalisée $\delta_{non\ pneu,i}(t)$ sont déterminées pour chacun d'eux et une moyenne de toutes les distances $\delta_{pneu}(t)$ et $\delta_{non\ pneu,i}(t)$ est trouvée pour obtenir une distance moyenne et un passage à la phase de ventilation suivante est réalisé quand la distance combinée est égale à 0.

**11.** Appareil selon la revendication 10, dans lequel la distance combinée est déterminée à partir de l'équation :

$$K(t) = \sum_{i=1}^{n} \frac{Q_{non\ pneu,i}}{\sum_{j=1}^{n} Q_{non\ pneu,j} + Q_{pneu}} \cdot \delta_{non\ pneu,i}(t) + \frac{Q_{pneu}}{\sum_{j=1}^{n} Q_{non\ pneu,j} + Q_{pneu}} \cdot \delta_{pneu}(t),$$

où $Q_{pneu}$ est un indicateur de qualité pour le signal pneumatique pour l'activité de respiration et $Q_{non\ pneu,i}$ est un indicateur de qualité pour le signal non pneumatique pour l'activité de respiration $u_{non\ pneu,i}(t)$ (i = 1, ..., n).

**12.** Appareil selon une quelconque revendication précédente, comprenant une machine de ventilation ayant une unité de ventilation ou une alimentation en gaz de respiration sous pression contrôlable, ayant une unité de contrôle qui est agencée pour contrôler l'unité de ventilation ou l'alimentation en gaz de respiration sous pression pour réaliser des phases successives d'inspiration et d'expiration, et des capteurs, pour détecter ledit signal pneumatique pour l'activité de respiration $u_{pneu}(t)$ et ledit signal non pneumatique pour l'activité de respiration $u_{non\ pneu}(t)$, qui sont reliés à l'unité de contrôle pour transmettre leurs signaux de mesure.

**Fig. 1**

Fig. 2

**Fig. 3**

Fig. 4

Fig. 5

## EP 2 465 560 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 102007062214 B3
- WO 2008131798 A1
- US 6588423 B1
- WO 9943374 A

### Non-patent literature cited in the description

- **MERLETTI R ; PARKER P.A.** Electromyography, Physiology, Engineering and Noninvasive Applications. IEEE Press, Wiley Interscience, 2004, 139 ff